# EUROPEAN PATENT APPLICATION

(11) **EP 2 777 510 A1**
(43) Date of publication of application: **17.09.2014**
(21) Application number: 14159912.6
(22) Date of filing: 14.03.2014
(51) Int. Cl.: A61B 17/02

(54) **Surgical retractor support system**

(30) Priority: 14.03.2013 US 201361782132 P
(71) Applicant: Tracto LLC, Noblesville, IN 46060 (US)
(72) Inventor: A. Gerdts, Zachary, Indianapolis IN 46256 (US); J. Shives, Jeremiah, Noblesville IN 46060 (US); Sieber, Jon, Indianapolis, IN 46236 (US); Kamradt, Brian, Indianapolis, IN 46217 (US); Davidson, Kyle, Noblesville, IN 46062 (US)
(74) Representative: Delorme, Nicolas

(57) **Abstract**

A surgical retractor support system includes a body extending from a first end to a second end through an intermediate portion, and a surgical retractor retaining configuration arranged relative to the body. The surgical retractor retaining configuration including an opening configured and disposed to receive a handle end of a surgical retractor.

## Description

### CROSS REFERENCE TO RELATED APPLICATIONS

This application is a non-provisional of U.S. Provisional Application No. 61/782,132 filed March 14, 2013, the disclosure of which is incorporated by reference herein in its entirety.

### BACKGROUND OF THE INVENTION

In the field of medicine, improvements are regularly made that improve outcomes and/or better manage patient needs. Other areas sometimes are neglected however and tend to not gain the efficiencies and benefits that improvements would bring. For instance, some conventional surgical instruments, such as surgical retractors, are primarily designed for function and are not generally ergonomically designed for the comfort or health benefits of the users.

### BRIEF DESCRIPTION OF THE INVENTION

Disclosed is a surgical retractor support system including a body extending from a first end to a second end through an intermediate portion, and a surgical retractor retaining configuration arranged relative to the body. The surgical retractor retaining configuration includes an opening configured and disposed to receive a handle end of a surgical retractor.

Also disclosed is a surgical retractor system including a surgical retractor extending from an operational end to a handle end, and a surgical retractor support system including a body extending from a first end to a second end through an intermediate portion, and a surgical retractor retaining configuration arranged relative to the body. The surgical retractor retaining configuration includes an opening receiving the handle end of the surgical retractor.

### BRIEF DESCRIPTION OF THE DRAWINGS

The following descriptions should not be considered limiting in any way. With reference to the accompanying drawings, like elements are numbered alike:
FIG. 1 is a side view of a surgical retractor system including a surgical retractor and a surgical retractor support, in accordance with an exemplary embodiment;
FIG. 2 is a perspective view of the surgical retractor support system of FIG. 1;
FIG. 3 is a perspective view of the surgical retractor support system of FIG. 1;
FIG. 4 is a perspective view of a surgical retractor support including a surgical retractor clamping assembly, in accordance with an aspect of an exemplary embodiment;
FIG. 5 is cross-sectional side view of a surgical retractor system including a surgical retractor clamping assembly, in accordance with another aspect of an exemplary embodiment;
FIG. 6 is a perspective view of a surgical retractor support including a surgical retractor clamping assembly including a lever mechanism shown in a latched configuration, in accordance with an exemplary embodiment;
FIG. 7 is a perspective view of a lever mechanism of the surgical retractor clamping assembly of FIG. 6;
FIG. 8 is a perspective view of the lever mechanism of FIG. 6 shown in an un-latched configuration;
FIG. 9 is a perspective view of a lever mechanism, in accordance with another aspect of an exemplary embodiment;
FIG. 10 is a cross sectional side view of the lever mechanism of FIG. 9; and
FIG. 11 is an exploded view of a surgical retractor support in accordance with another aspect of an exemplary embodiment.

### DETAILED DESCRIPTION OF THE INVENTION

A detailed description of one or more embodiments of the disclosed apparatus and method are presented herein by way of exemplification and not limitation with reference to the Figures. Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including", "comprising", or "having" and variations thereof herein is meant to encompass the items listed thereafter and equivalents thereof as well as additional items. Unless specified or limited otherwise, the terms "attached", "mounted", "connected", "supported", and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the invention. Various modifications to the illustrated embodiments will be readily apparent to those skilled in the art, and the generic principles therein can be applied to other embodiments and applications without departing from the embodiments of the invention. Thus, embodiments of the invention are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of embodiments of the invention. Skilled artisans will recognize the examples provided herein have many useful alternatives that fall within the scope of embodiments of the invention.

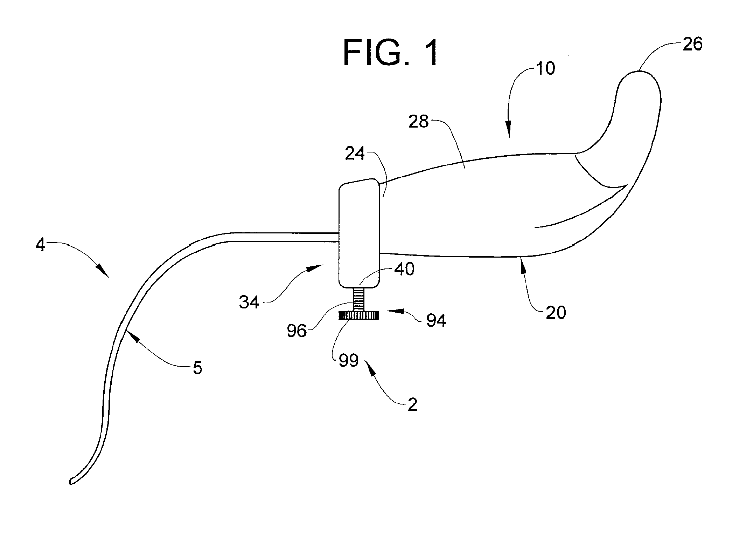

With initial reference to FIG. 1, a surgical retractor system, in accordance with an exemplary embodiment, is indicated generally at 2. Surgical retractor system 2 includes a surgical retractor 4 shown in the form of a retractor 5 coupled to a surgical retractor support system 10. In a number of embodiments, surgical retractor 4 may possess a handle having an aspect ratio of greater than 1 and the surgical retractor support system 10 will interact with that handle.

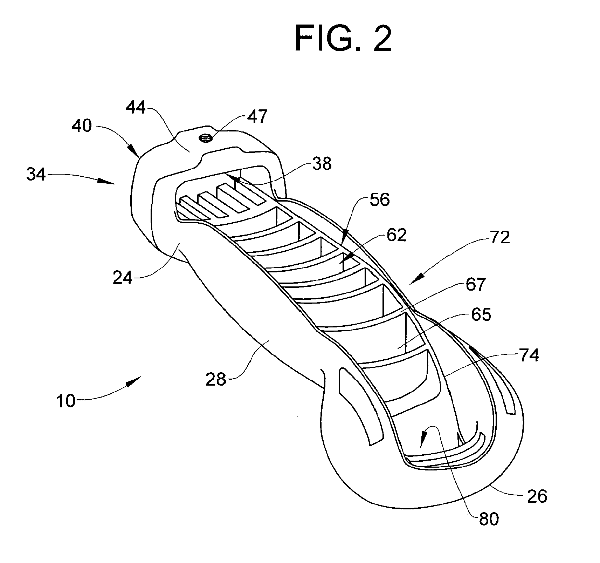

In accordance with an exemplary embodiment, surgical retractor support system 10 includes a body 20 extending from a first end 24 to a second end 26 through an intermediate portion 28. A surgical retractor retaining configuration 34 is arranged relative to the body 20 so that interaction with, and retention of, a surgical retractor 4 may be effected. As illustrated in FIG. 1, surgical retractor retaining configuration 34 is arranged at first end 24 of body 20. As shown in FIG. 2, surgical retractor retaining configuration 34 includes an opening 38 defined by a perimetrical wall 40. A base 44 having a threaded opening 47 is formed in perimetrical wall 40. Body 20 includes an inner surface 56 which, in the exemplary embodiment shown, may be defined, at least in part, by a plurality of ribs 62. Inner surface 56 may be externally exposed. Specifically, ribs 62 extend from a first end or base 65 coupled to body 20 to a second, cantilevered end 67 that defines a portion of inner surface 56. Of course, it should be understood that inner surface 56 may also be a continuous surface that extends between first and second ends 24 and 26. Inner surface 56 includes a contour 72 that, in the exemplary embodiment shown, takes the form of a curvilinear contour 74. Contour 72 may take on a variety of other geometries. Body 20 is also shown to include a recessed portion 80 provided at second end 26. Recessed portion 80 may receive a portion of surgical retractor 4.

Body 20 is shaped to allow multiple interface or gripping configurations. Body 20 may have a single gripping portion defined by intermediate portion 28 alone or second end 26 alone or may have multiple gripping portions including intermediate portion 28 and second end 26. For example, second end 26 can be gripped or used to provide additional leverage to surgical retractor 4 or can be gripped on its own similarly to a vehicular center console gearshift. Further, body 20 may be formed from multiple elements that may move telescopically relative to one another. In addition, inner surface 56 is configured to accept a wide array of surgical retractor handle configurations.

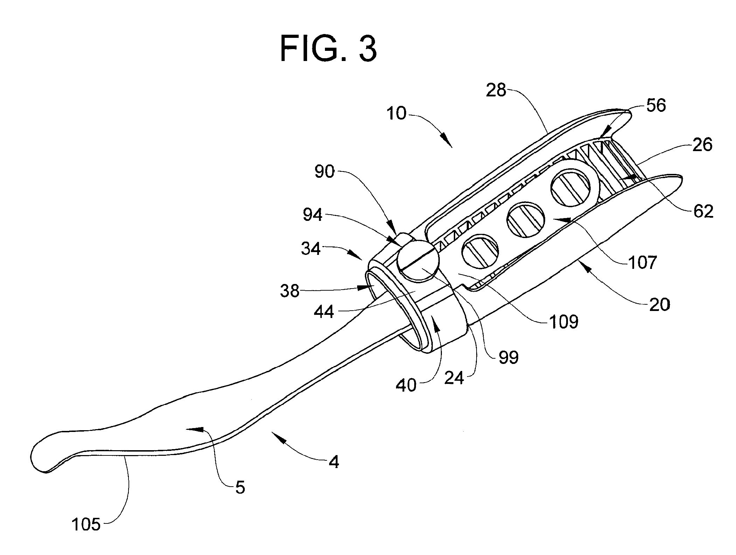

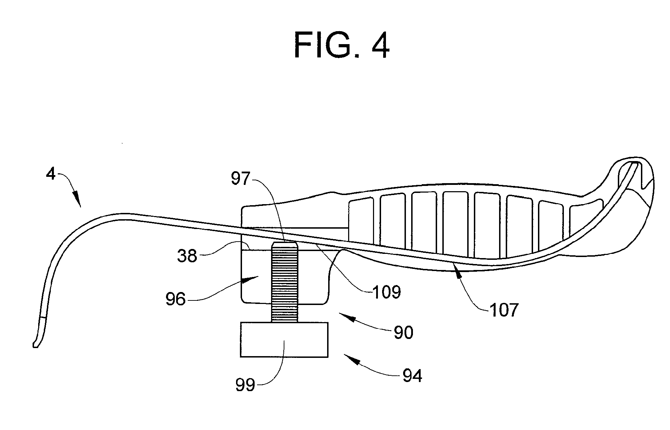

In further accordance with an exemplary embodiment, surgical retractor support system 10 includes a surgical retractor clamping assembly 90. In accordance with an aspect of an exemplary embodiment illustrated in FIGs. 3-4, surgical retractor clamping assembly 90 takes the form of a mechanical fastener 94. Mechanical fastener 94 is configured to allow a user to secure surgical retractor support system 10 to surgical retractor 4 rapidly and reliably, including against a tensile moment imposed upon the surgical retractor support system 10 relative to surgical retractor 4. Mechanical fastener 94, in the illustrated embodiment, includes a threaded portion 96 that cooperates with threaded opening 47 to support surgical retractor clamping assembly 90 in surgical retractor retaining configuration 34. Threaded portion 96 includes a terminal end portion 97 that is selectively moved into engagement with surgical retractor 4 by rotating, for example, a head end 99 of mechanical fastener 94. More specifically, surgical retractor 4 extends from an operational end 105 to a handle end 107. Handle end 107 includes a non-circular cross-section having a surface 109. Head end 99 of mechanical fastener 94 may be manipulated to move terminal end portion 97 against surface 109 thereby clamping handle end 107 in surgical retractor retaining configuration 34. Surface 109 may be provided with one or more recesses (not shown) configured to interact with terminal end portion 97 to further constrain movement of surgical retractor 4 relative to surgical retractor support system 10.

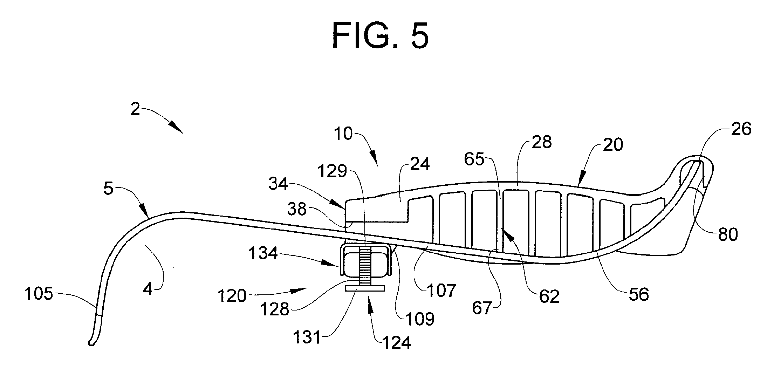

FIG. 5 illustrates a surgical retractor clamping assembly 120, in accordance with another aspect of an exemplary embodiment. Surgical retractor clamping assembly 120 in the illustrated embodiment includes a mechanical fastener 124 having a threaded portion 128 terminating at a terminal end portion 129 and a head end 131. A plate element 134 is provided at terminal end portion 129. Plate element 134 is selectively moved into engagement with surface 109 to retain handle end 107 in surgical retractor retaining configuration 34. Plate element 134 distributes a compression force applied by end portion 129 over a larger area of surface 109 to provide an enhanced clamping and retention capacity.

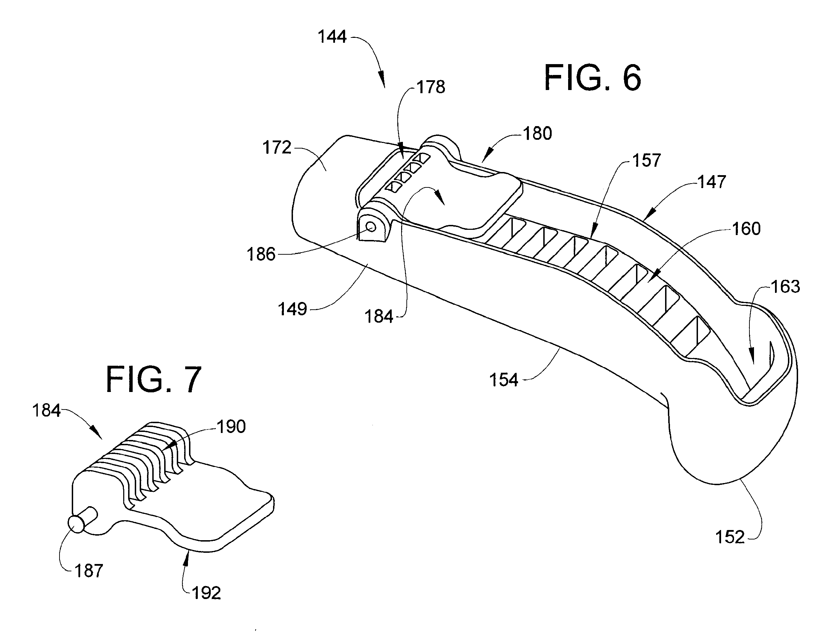

FIG. 6 illustrates a surgical retractor support system 144, in accordance with another aspect of an exemplary embodiment. Surgical retractor support system 144 includes a body 147 extending from a first end 149 to a second end 152 through an intermediate portion 154. Body 147 includes an inner surface 157 which, in a manner similar to that discussed above, may be defined, at least in part, by a plurality of ribs 160. Inner surface 157 may also be externally exposed. Second end 152 includes a recessed portion 163 that may receive a portion of handle end 107 of surgical retractor 4. First end 149 includes an opening 169 defined by a perimetrical wall 172. Opening 169 receives handle end 107 of surgical retractor 4.

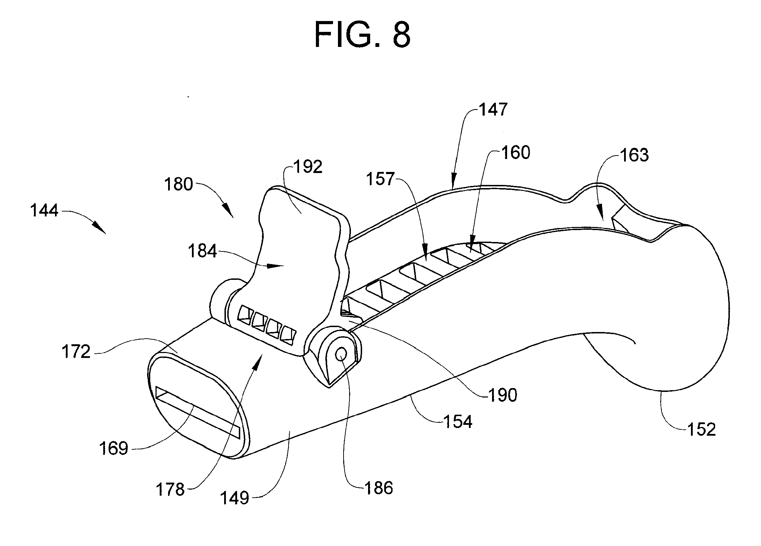

Surgical retractor support system 144 further includes a surgical retractor retaining configuration 178 arranged relative to body 147. In the illustrated embodiment, surgical retractor retaining configuration 178 includes a surgical retractor clamping assembly 180 mounted in first end 149. In the illustrated embodiment, surgical retractor clamping assembly 180 is arranged at first end 149. Surgical retractor clamping assembly 180 is shown in the form of a lever mechanism 184 pivotally mounted at first end 149. As shown in FIG. 7, lever mechanism 184 includes a first pin element 186 (FIG. 6) and a second pin element 187 that define a pivoting axis for lever mechanism 184. In the illustrated embodiment, lever mechanism 184 also includes a plurality of cam elements 190 that selectively apply a compressive force to surface 109 of surgical retractor 4 and an actuator 192. However, it should be understood, that the number of cam elements 190 may vary. In operation, actuator 192 may be manipulated to pivot lever mechanism 184 between a first or un-latched position, shown in FIG. 8, allowing for the insertion and/or removal of surgical retractor 4, and a latched configuration, shown in FIG. 6, in which cam elements 190 apply the compressive force to surface 109 to retain surgical retractor 4 in surgical retractor support system 144. It is to be appreciated that the mechanism could also be positioned to hold surgical retractor 4 from a side of the handle rather than surface 109, if desired.

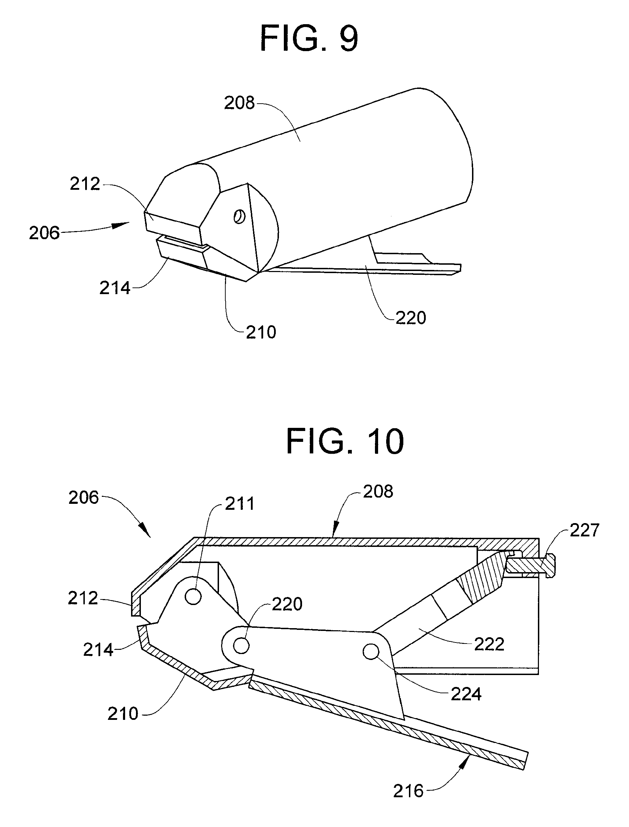

FIGs. 9-10 illustrate a lever mechanism 206, in accordance with another aspect of an exemplary embodiment. Lever mechanism 206 includes a stationary member 208 connected to a moveable member 210 through a hinge 211. Stationary member 208 includes a stationary jaw 212 and moveable member 210 includes a moveable jaw 214. Moveable member 210 is pivoted relative to stationary member 208 creating a clamping force between stationary jaw 212 and moveable jaw 214. Lever mechanism 206 includes a latch handle 216 pivotally connected to moveable member 210 through a hinge 220. A linking bar 222 is coupled between stationary member 208 and latch handle 216 through an over-center hinge 224. Linking bar 222 includes an adjustable pivot 227 at stationary member 208 which may be manipulated to accommodate surgical retractors having differing thicknesses.

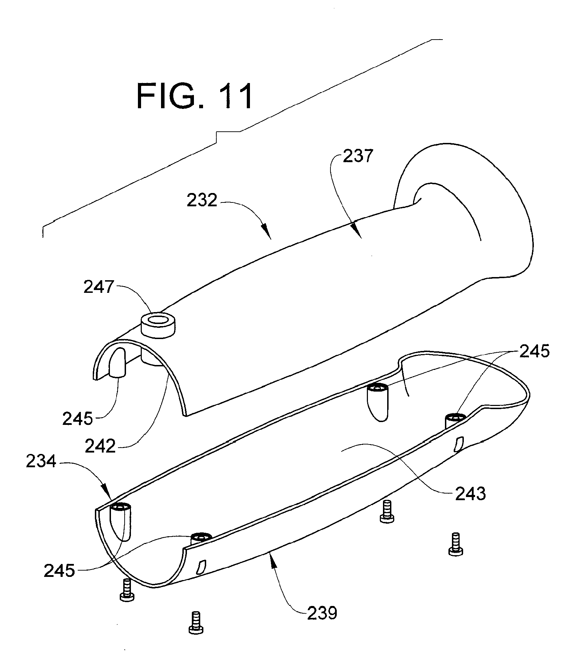

FIG. 11 illustrates a surgical retractor support system 232, in accordance with another exemplary embodiment. Surgical retractor support system 232 includes a body 234 formed from a first shell section 237 and a second shell section 239 that may be joined about lever mechanism 206. First and second shell sections 237 and 239 include corresponding first and second inner surfaces 242 and 243 that are not externally exposed when first shell section 237 is joined with second shell section 239. Each inner surface 242 and 243 includes one or more mounting elements 245. Mounting elements 245 register, one with another, and may receive mechanical fasteners (not shown) that join first shell section 237 with second shell section 239. First shell section 237 may also include a mounting member 247 that aligns with an opening (not separately labeled) in lever mechanism 206. A mechanical fastener (not shown) may be used to join lever mechanism 206 to surgical retractor support system 232. Surgical retractor support system 232 may be provided with a surgical retractor retaining configuration 34 such as described above.

At this point it should be understood that the exemplary embodiments describe a surgical retractor support system that provides a user with a more ergonomic gripping surface for a surgical retractor. Further, while shown as having an additional gripping surface at the second end, the surgical retractor support system may also be formed without terminal contours that aid in gripping/manipulating a surgical retractor. It should be also understood that the surgical retractor support system may be formed as a single use, e.g., disposable unit, or may be formed from a material that is configured to withstand a sterilization process. Regardless of form or material, the surgical retractor support system provides surgeons and/or surgical nurses/staff with a more ergonomic gripping surface on a surgical retractor that is more readily manipulated and also relieves stress during surgery. Specifically, a surgical retractor is often under a tensile force for prolonged periods to provide access to a surgical field. The surgical retractor support system, in accordance with exemplary embodiments, enables medical support personnel to apply the tensile force to the surgical retractor with reduced fatigue.

While the invention has been described with reference to an exemplary embodiment or embodiments, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the claims.

## Claims

1. A surgical retractor support system comprising:
a body extending from a first end to a second end through an intermediate portion; and
a surgical retractor retaining configuration arranged relative to the body, the surgical retractor retaining configuration including an opening configured and disposed to receive a handle end of a surgical retractor.

2. The surgical retractor support system according to claim 1, wherein the intermediate portion includes at least one inner surface, the at least one inner surface being externally exposed between the first end and the second end.

3. The surgical retractor support system according to claim 2, wherein the at least one inner surface includes a contour.

4. The surgical retractor support system according to any one of claims 1 to 3, wherein the second end includes a recessed portion configured and disposed to receive a handle end of a surgical retractor.

5. The surgical retractor support system according to any one of claims 1 to 4 further comprising: a surgical retractor clamping assembly arranged in the surgical retractor retaining configuration, wherein the surgical retractor clamping assembly comprises a mechanical fastener having a threaded portion including an end portion and a head end, the threaded portion being configured to threadingly engage the surgical retractor retaining configuration with the end portion extending into the opening.

6. The surgical retractor support system according to claim 5, further comprising: a plate element arranged at the end portion of the mechanical fastener.

7. The surgical retractor support system according to any one of claims 1 to 6, wherein the surgical retractor retaining configuration includes a surgical retractor clamping assembly mounted at the first end, the surgical retractor clamping assembly including a lever mechanism.

8. The surgical retractor support system according to claim 7, wherein the lever mechanism includes a stationary jaw and a moveable jaw operatively coupled to a latch handle through a hinge.

9. The surgical retractor support system according to any one of claims 1 to 8, wherein the body includes at least one gripping portion.

10. The surgical retractor support system according to claim 9, wherein the at least one gripping portion comprises the intermediate portion and the second end.

11. A surgical retractor system comprising:
a surgical retractor extending from an operational end to a handle end; and
a surgical retractor support system including a body extending from a first end to a second end through an intermediate portion, and a surgical retractor retaining configuration arranged relative to the body, the surgical retractor retaining configuration including an opening receiving the handle end of the surgical retractor.

12. The surgical retractor system according to claim 11, wherein the intermediate portion includes at least one inner surface, the at least one inner surface being externally exposed between the first end and the second end.

13. The surgical retractor system according to claim 11 or claim 12, further comprising: a surgical retractor clamping assembly arranged in the surgical retractor retaining configuration, wherein the surgical retractor clamping assembly comprises a mechanical fastener having threaded portion including an end portion and a head end, the threaded portion being configured to threadingly engage the surgical retractor retaining configuration with the end portion extending into the opening and engaging the handle end of the surgical retractor.

14. The surgical retractor system according to claim 13, wherein the surgical retractor clamping assembly includes a lever mechanism.

15. The surgical retractor system according to any one of claims 11 to 14, wherein the body includes at least one gripping portion.
